# EUROPEAN PATENT APPLICATION

(11) **EP 4 420 698 A1**
(43) Date of publication of application: **28.08.2024**
(21) Application number: 23305248.9
(22) Date of filing: 24.02.2023
(51) Int. Cl.: A61M 5/315, A61M 5/20, A61M 5/32

(54) **STABILIZED PLUNGER BODY FOR AUTOINJECTOR**

(71) Applicant: Becton, Dickinson and Company, Franklin Lakes, NJ 07417-1880 (US)
(72) Inventor: BESSON, Nicolas, 38650 Treffort (FR); PLOUVIER, Adrien, 38400 SAINT MARTIN D'HERES (FR)
(74) Representative: Germain Maureau

(57) **Abstract**

Provided herein is a plunger subassembly for a drug delivery device, including a plunger body having a proximal end, a distal end, and a sidewall therebetween, the plunger body comprising a head at the proximal end thereof, the head defining a passage therethrough, and a plunger rod having a proximal end and a distal end, the plunger rod comprising a clip at the proximal end thereof, wherein the clip is configured to be received within the opening of the plunger body head and to limit axial displacement between the plunger rod and the plunger body, and to limit radial deflection of the plunger rod.

## Description

### BACKGROUND OF THE INVENTION

### Field of the Invention

The present disclosure relates generally to a drug delivery device and, more specifically, to an auto-injector.

### Description of Related Art

Various types of automatic injection devices have been developed to allow drug solutions and other liquid therapeutic preparations to be administered by untrained personnel or to be self-injected. Generally, these devices include a reservoir that is pre-filled with the liquid therapeutic preparation, and some type of automatic needle-injection mechanism that can be triggered by the user. Many of these devices, such as auto-injectors, are designed so that the reservoir, such as a pre-filled syringe, is assembled into the device during assembly of the device. In addition to automatically deploying the needle-injection mechanism, many drug delivery devices also automatically shield the needle after use of the device to prevent any unintended contact with the needle.

In some automatic injection devices, manufacturing can require use of multiple component subassemblies, such as plungers. It is desirable to limit unwanted movement/displacement of various components, while allowing for a minor amount of desired displacement, to account for manufacturing tolerances, to ensure proper operation of the device and accurate delivery of therapeutic substances.

### SUMMARY OF THE INVENTION

Provided herein is a plunger subassembly for a drug delivery device, including a plunger body having a proximal end, a distal end, and a sidewall therebetween, the plunger body comprising a head at the proximal end thereof, the head defining a passage therethrough, and a plunger rod having a proximal end and a distal end, the plunger rod comprising a clip at the proximal end thereof, wherein the clip is configured to be received within the opening of the plunger body head and to limit axial displacement between the plunger rod and the plunger body, and to limit radial deflection of the plunger rod.

Also provided herein is a drug delivery device having a housing having a proximal end and a distal end, a syringe including a barrel, a stopper, and a needle arranged at a distal end of the syringe, at least a portion of the syringe positioned within the housing, a drive assembly including a drive member and a plunger assembly, the plunger assembly including a plunger body having a proximal end, a distal end, and a sidewall therebetween, the plunger body having a head at the proximal end thereof, the head defining an opening therethrough and a plunger rod having a proximal end and a distal end, the plunger rod including a clip at the proximal end thereof, wherein the clip is configured to be received within the opening of the plunger body head and to limit axial displacement between the plunger rod and the plunger body, and to limit radial deflection of the plunger rod, where the drive assembly is configured to move the stopper within the barrel upon actuation of the drive assembly, at least a portion of the drive assembly positioned within the housing, and a needle cover having a pre-use position where the needle is positioned within the needle cover, an actuation position where the needle cover has been shifted proximally, thereby allowing the drive assembly to be actuated, and a post-use position where the needle is positioned within the needle cover.

### BRIEF DESCRIPTION OF THE DRAWINGS

**FIG. 1** is a perspective view of a drug delivery device according to non-limiting embodiments described herein;
**FIG. 2** is a cross-sectional view of a drug delivery device according to non-limiting embodiments described herein;
**FIG. 3** is a perspective view of a plunger assembly for a drug delivery device according to non-limiting embodiments described herein;
**FIGS. 4A and 4B** are perspective and enlarged views of a plunger assembly for a drug delivery device according to non-limiting embodiments described herein;
**FIGS. 5A and 5B** are perspective and enlarged views of a plunger assembly for a drug delivery device according to non-limiting embodiments described herein;
**FIG. 6** is an enlarged view of a plunger assembly for a drug delivery device according to non-limiting embodiments described herein; and
**FIG. 7** is an enlarged view of a plunger assembly for a drug delivery device according to non-limiting embodiments described herein.

### DESCRIPTION OF THE INVENTION

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume various alternative variations, except where expressly specified to the contrary. It is also to be understood that the specific devices illustrated in the attached drawings, and described in the following specification, are simply exemplary embodiments of the invention. Hence, specific dimensions and other physical characteristics related to the embodiments disclosed herein are not to be considered as limiting.

It should be understood that any numerical range recited herein is intended to include all values and sub-ranges subsumed therein. For example, a range of" 1 to 10" is intended to include all sub-ranges between (and including) the recited minimum value of 1 and the recited maximum value of 10, that is, having a minimum value equal to or greater than 1 and a maximum value of equal to or less than 10.

International Patent Application Publication Nos. WO 2020/173991, WO 2020/173992, WO 2020/173993, WO 2020/173994, and WO 2020/173995 are incorporated herein by reference in their entirety.

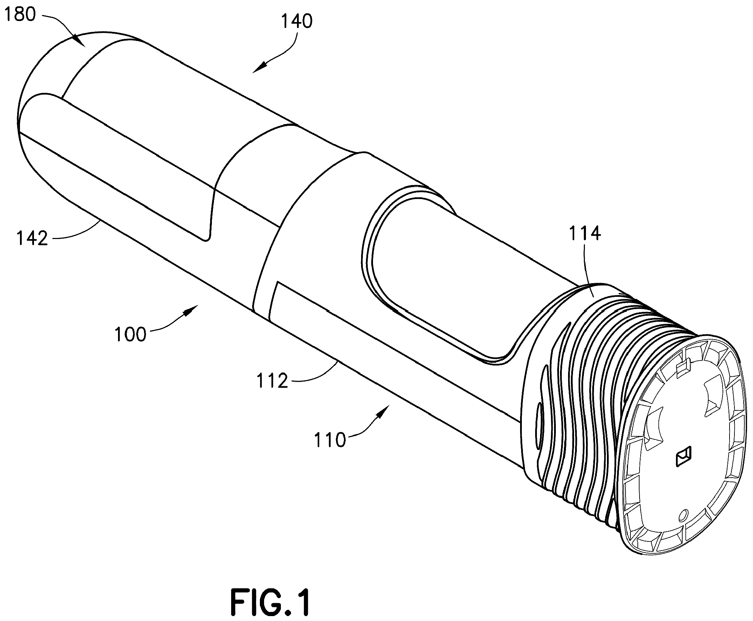

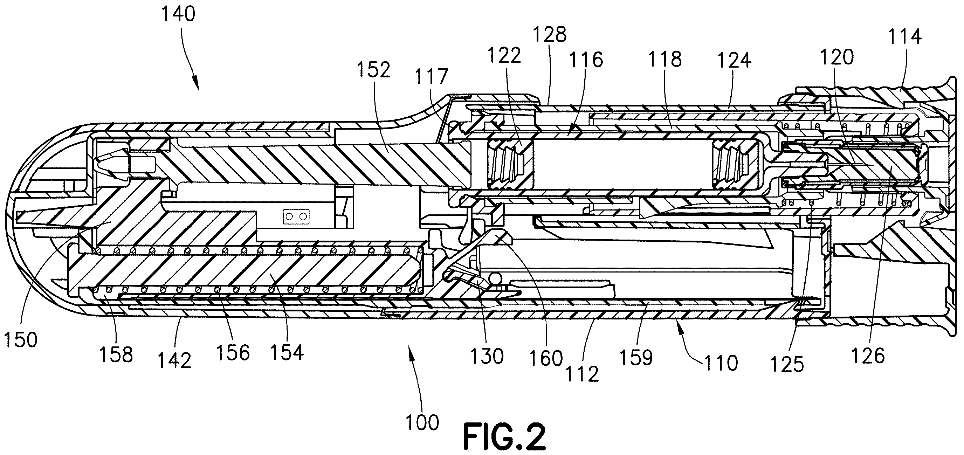

Turning to FIGS. 1 and 2, shown is a drug delivery device 100. Drug delivery device 100 may include a first subassembly 110, a second subassembly 140, and a syringe 116. The first subassembly 110 may include lower housing shell 112, a cap 114, a syringe holder 118, a needle cover 124, and a cassette body 128. The second subassembly 140 may include a drive assembly including a plunger body 150, the plunger body 150 including a plunger rod 152 and a spring guide member 154 including a drive member 156 and a drive opening 158. Second subassembly 140 may further include a motor body 159, a lever actuation member 130, and an upper housing shell 142. The syringe 116 may be received by the syringe holder 118 and includes a barrel 117, a stopper 122, a needle 120, and a rigid needle shield (RNS) 126. Although an RNS is utilized, other suitable needle shield arrangements may be utilized. The lower housing shell 112, the cassette body 128, and the upper housing shell 142 generally form a housing for receiving the various components of the drug delivery device 100, although other suitable housing arrangements may be utilized. The first subassembly 110 and the second subassembly 140 may be secured to each other during assembly by a locking clip, although other suitable arrangements may be utilized. The drug delivery device 100 may be an auto-injector, although the features described herein may be incorporated into other suitable drug delivery devices.

Drug delivery device 100 is configured to automatically deliver a dose of medicament from the syringe 116 to a patient upon actuation of the device 100. More specifically, upon actuation of the drug delivery device 100, the drive assembly is configured to engage the stopper 122 of the syringe 116, displace the syringe 116 such that the needle 120 pierces the skin of the patient, and displace the stopper 122 within the barrel 117 of the syringe 116 to deliver the medicament within the barrel 117. The drug delivery device 100 includes a storage position, a pre-use position, an actuation position, an injection position, and a post-use position, as described in International Patent Application Publication Nos. WO 2020/173991, WO 2020/173992, WO 2020/173993, WO 2020/173994, and WO 2020/173995.

Needle cover 124 is configured to shield the needle 120 of the syringe 116 from the patient when the device 100 is in the pre-use and the post-use positions. In particular, the needle cover 124 is moveable between a pre-use position, an actuation position, and a post-use positon, with a spring 125 biasing the needle cover 124 towards the pre-use position and the post-use position. The spring 125 may be positioned between the needle cover 124 and the syringe holder 118, although other suitable arrangements may be utilized. The lever actuation member 130 is moveable between a locked position where movement of the drive assembly is prevented and a released position where movement of the drive assembly is allowed. More specifically, the lever actuation member 130 is rotatable about a rotation axis between the locked position and the released position. When the lever actuation member 130 is in the locked position, the lever actuation member 130 is engaged with the motor body 159 and the drive assembly to prevent movement of the drive assembly.

When the lever actuation member 130 is in the released position, which may be achieved by pressing the needle cover 124 onto the patient's skin at the site of injection, shifting the needle cover 124 proximally into the lower housing shell 112, the lever actuation member 130 is disengaged from the motor body 159 thereby allowing movement of the drive assembly toward the syringe 116. The rotation axis of the lever actuation member 130 extends perpendicular to a longitudinal axis of the device 100, although other suitable arrangements may be utilized.

Referring again to FIGS. 1 and 2, the drive assembly includes a plunger body 150 having a plunger rod 152 and a drive member 156. The drive member 156 may be a compression spring received within a drive opening 158 defined by the plunger body 150, although other suitable drive members may be utilized, including, but not limited to, compressed gas, an electric motor, hydraulic pressure, other types of springs, etc. The drive member 156 engages the plunger body 150 and the motor body 159 and biases the plunger body 150 in a direction extending from the second subassembly 140 toward the first subassembly 110. The plunger body 150 defines a lever opening that receives the lever actuation member 130 and defines the rotation axis of the lever actuation member 130. The lever actuation member 130 prevents movement of the plunger body 150 when the lever actuation member 130 is in the locked position through engagement of the lever actuation member 130 with the motor body 159. Upon rotation of the lever actuation member 130 from the locked position to the released position, the lever actuation member 130 is disengaged from the motor body 159 thereby allowing the drive member 156 to move the plunger body 150 and the plunger rod 152 toward the first subassembly 110. The plunger rod 152 and the drive member 156 are spaced from and parallel to each other and extend in a longitudinal direction of the device 100.

The drive assembly further includes a spring guide member 154 secured to the upper housing shell 142 and received within the drive opening 158 of the plunger body 150. The drive member 156 is received by the spring guide member 154 such that the drive member 156 is positioned between the plunger body 150 and the spring guide member 154. The drive assembly may also include a plunger rod cover that receives the plunger rod 152 of the plunger body 150. The plunger rod cover may be configured to guide insertion of the plunger rod 152 into the barrel 117 of the syringe 116 and engage the stopper 122 to dispense the medicament from the barrel 117 of the syringe 116. The plunger rod cover and the plunger rod 152 may be formed integrally or formed as separate components.

The plunger body 150 of the drive assembly may also include an audio indicator member 160 configured to provide an audible indication to a user when the device 100 transitions to the post-use position. The audio indicator member 160 may be configured to engage one or more ribs of the cassette body 128 when the device 100 is in the injection position thereby deflecting the audio indicator member 160. When the drug delivery device 100 transitions from the injection position to the post-use position, the audio indicator member 160 may disengage from the rib(s) of the cassette body 128 and contact the lower housing shell 112 to provide an audible click, although the audio indicator member 160 could also contact other suitable portions of the device 100 to provide the audible indicator.

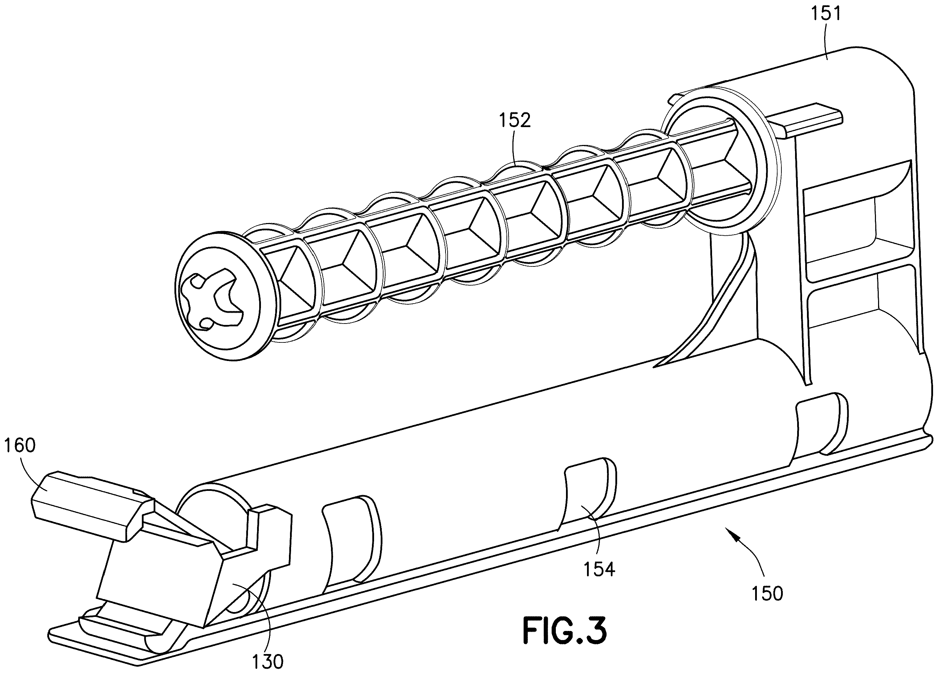

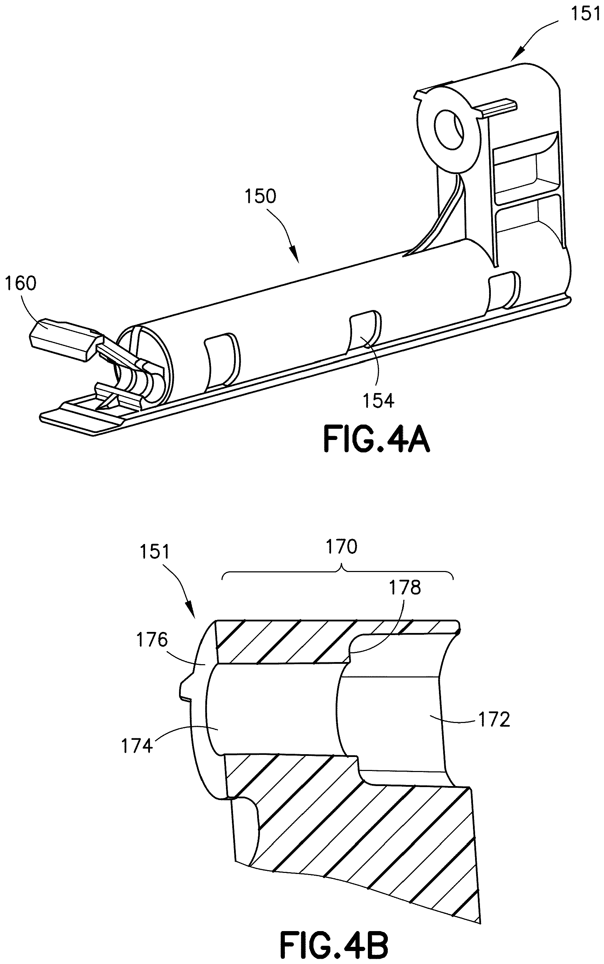

Turning to FIGS. 3-4B, shown is a non-limiting embodiment of a plunger assembly, including a plunger body 150 and plunger rod 152. Plunger rod 152 may be offset from plunger body 150, for example radially offset, as shown in FIGS. 3 and 4A. Plunger body 150 may include plunger body head 151, which may be configured to engage plunger rod 152. As shown in FIG. 4B, plunger body head 151 may define a passage 170 therethrough. In non-limiting embodiments, passage 170 may include a plurality of portions, including first portion 172 and second portion 174. First portion 172 and second portion 174 may have distinct diameters. For example, first portion 172 may be arranged proximally of second portion 174, and may have a first diameter. Second portion 174 may have a second diameter, and the first diameter may be larger than the second diameter. In non-limiting embodiments, a transition from first portion 172 to second portion 174 of passage 170 may include a shoulder 178 having a proximal-facing surface. In non-limiting embodiments, plunger body head 151 may include a distal-facing surface 176.

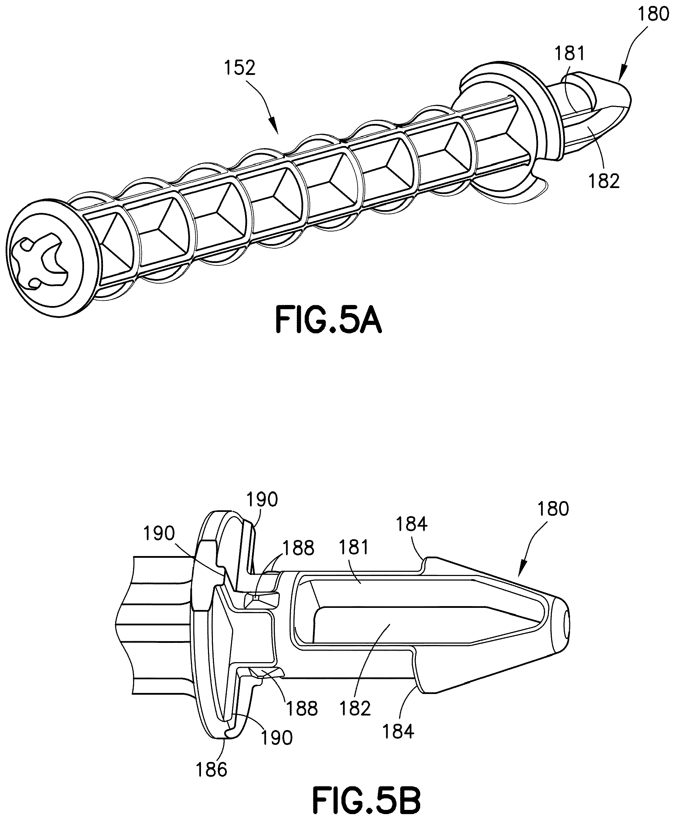

Turning to FIGS. 5A-5B, shown is plunger rod 152, including a plunger rod clip 180 arranged at a proximal end thereof. Clip 180 may allow for plunger rod 152 to couple with plunger body 150, through engagement of clip 180 with plunger rod head 151. Plunger rod clip 180 may include a plurality of sidewalls 181 defining a longitudinally-extending opening 182. By virtue of opening 182, and thickness of sidewalls 181, sidewalls 181 may be flexible, as will be discussed below. Accordingly, clip 180, including sidewalls 181, may be formed of a resilient yet flexible material, such as plastics. In non-limiting embodiments, clip 180 may be formed of polyoxymethylene-containing and/or polypropylene-containing materials. Sidewalls 181 may include one or more flange(s) 184, extending radially outward and defining a distally-facing surface. This distally-facing surface may be configured to engage the shoulder 178 (e.g., the proximally-facing surface thereof) at the transition between first portion 172 and second portion 174 of passage 170 through plunger body head 151.

With continuing reference to FIG. 5B, proximal end of plunger rod 152 may further include one or more radially-extending dots 188 arranged about the perimeter. In non-limiting embodiments, four dots 188 are arranged at 90° intervals about the perimeter of plunger rod 152. In non-limiting embodiments, dots 180 provide for a tight fit between plunger rod 152 and plunger body 150, reducing and/or preventing movement and unwanted noise, and/or facilitating assembly of second subassembly 140. In non-limiting embodiments, proximal end of plunger rod 152 further includes a base 186. Base 186 may extend radially outward from plunger rod 152, for example, base 186 may extend radially outward from plunger rod 152 a greater distance than sidewalls 181 and/or flange(s) 184. In non-limiting embodiments, base 186 includes one or more ribs 190 arranged thereon, for example on a proximal-facing surface thereof. As with dot(s) 188, a plurality of ribs 190, e.g., four ribs, may be arranged at 90° intervals about base 186.

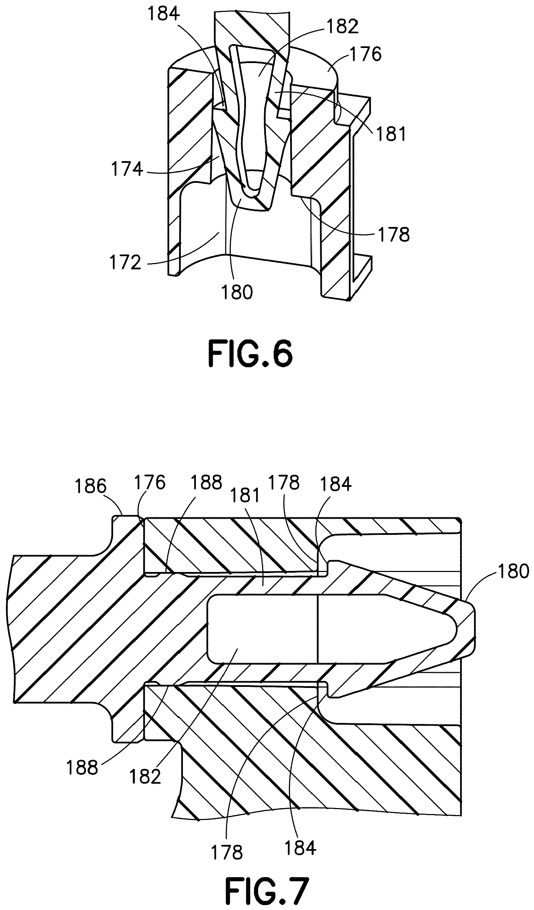

Turning to FIGS. 6 and 7, shown are steps of assembling a plunger assembly, including initial engagement between plunger rod 152 and plunger body 150, in particular plunger body head 151. As shown in FIG. 6, in a first step, clip 180 is inserted into second portion 174 of passage through plunger body head. In the illustrated non-limiting embodiment, second portion 174 has a smaller diameter than that of clip 180, in particular the diameter of clip 180 defined by flange(s) 184. Accordingly, engagement between flange(s) 184 and inner surface of second portion 174 of passage 170 causes an inward flex of flange(s) 184 and sidewalls 181. Turning to FIG. 7, as plunger rod 152 is moved proximally, flange(s) 184 pass the transition between first portion and second portion of passage, and enters first portion. Flange(s) 184 and sidewalls 181, which are resilient, return to their original orientation upon entering first portion 172 of passage 170, which has a larger diameter than second portion 174. Distal-facing surface of flange(s) 184 engage proximal facing surface of shoulder 178, thus locking clip 180 and plunger rod 152 in place. At the other end of passage 170, proximal-facing surface of base 186, of plunger rod 152 engages distal-facing surface 176 of plunger body head 151. In non-limiting embodiments, rib(s) 190 arranged on base 186 engage distal-facing surface 176 of plunger body head 151. The distance between base 186 and flange(s) 184 (optionally between rib(s) 190 and flange(s) 184) may be arranged to limit, or eliminate, relative axial movement between plunger rod 152 and plunger body head 151 once plunger assembly is assembled.

With continuing reference to FIG. 7, in non-limiting embodiments, clip 180 includes one or more dot(s) 188, extending radially outward from clip 180. Dot(s) 188 may interact with an inner surface of second portion 174 of passage 170. In non-limiting embodiments, inner surface of passage 170 does not include any divots to receive dot(s) 188. Arrangement of dot(s) 188 may allow for a small amount of angular displacement of plunger rod 152, relative to plunger body head 151, to account for manufacturing tolerances, thus allowing plunger rod 152 to slide within the barrel 117 of syringe 116 received within drug delivery device 100, even if barrel 117 is radially offset from the longitudinal axis of plunger rod 152 and/or plunger body 150.

Although the present disclosure has been described in detail for the purpose of illustration based on what is currently considered to be the most practical and preferred embodiments, it is to be understood that such detail is solely for that purpose and that the present disclosure is not limited to the disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A plunger subassembly for drug delivery device, comprising:
a plunger body having a proximal end, a distal end, and a sidewall therebetween, the plunger body comprising a head at the proximal end thereof, the head defining a passage therethrough; and
a plunger rod having a proximal end and a distal end, the plunger rod comprising a clip at the proximal end thereof,
wherein the clip is configured to be received within the opening of the plunger body head and to limit axial displacement between the plunger rod and the plunger body, and to limit radial deflection of the plunger rod.

2. The plunger body of claim 1, wherein the plunger body head is radially offset from the plunger body sidewall.

3. The plunger subassembly of claim 1 or claim 2, wherein the passage through the plunger body head comprises a proximal section having a first diameter and a distal section having a second diameter.

4. The plunger subassembly of claim 3, wherein the first diameter is larger than the second diameter.

5. The plunger subassembly of claim 4, wherein a transition from the first diameter to the second diameter comprises a shoulder having a proximal-facing surface.

6. The plunger subassembly of any of claims 1-5, wherein the clip comprises a proximal end, a distal end, a plurality of sidewalls defining an opening, and a base arranged at the distal end.

7. The plunger assembly of claim 6, wherein the sidewalls are compressible.

8. The plunger subassembly of any of claims 1-7, wherein the proximal end of the clip defines a plurality of radially-outward extending protrusions.

9. The plunger assembly of claim 8, wherein the plurality of radially-outward extending protrusions each comprise an engagement surface, the engagement surfaces configured to engage the proximal-facing surface of the shoulder of the plunger body head.

10. The plunger assembly of any of claims 1-9, wherein the distal end of the clip comprises a plurality of outwardly-extending protrusions configured to engage an interior surface of the opening in the plunger body head.

11. The plunger assembly of any of claims 1-10, wherein the distal end of the clip comprises a base configured to engage a distal-facing surface of the plunger body head.

12. The plunger assembly of claim 11, wherein the base comprises a plurality of proximally-extending ribs configured to engage a distal-facing surface of the plunger body head.

13. A drug delivery device comprising:
a housing having a proximal end and a distal end;
a syringe comprising a barrel, a stopper, and a needle arranged at a distal end of the syringe, at least a portion of the syringe positioned within the housing;
a drive assembly comprising a drive member and a plunger assembly comprising:
a plunger body having a proximal end, a distal end, and a sidewall therebetween, the plunger body comprising a head at the proximal end thereof, the head defining an opening therethrough; and
a plunger rod having a proximal end and a distal end, the plunger rod comprising a clip at the proximal end thereof, wherein the clip is configured to be received within the opening of the plunger body head and to limit axial displacement between the plunger rod and the plunger body, and to limit radial deflection of the plunger rod, wherein
the drive assembly configured to move the stopper within the barrel upon actuation of the drive assembly, at least a portion of the drive assembly positioned within the housing; and
a needle cover having a pre-use position where the needle is positioned within the needle cover, an actuation position where the needle cover has been shifted proximally, thereby allowing the drive assembly to be actuated, and a post-use position where the needle is positioned within the needle cover.

14. The drug delivery device of claim 13, wherein
the plunger body head is radially offset from the plunger body sidewall;
the opening through the plunger body head comprises a proximal section having a first diameter and a distal section having a second diameter, the first diameter being smaller than the second diameter, and wherein a transition from the first diameter to the second diameter comprises a shoulder having a proximal-facing surface.

15. The drug delivery device of claim 14, wherein
the clip comprises a proximal end, a distal end, a plurality of compressible sidewalls defining an opening, and a base arranged at the distal end;
the proximal end of the clip defines a plurality of radially-outward extending protrusions each comprising an engagement surface, the engagement surfaces configured to engage the proximal-facing surface of the shoulder of the plunger body head; and
the distal end of the clip comprises:
a plurality of outwardly-extending protrusions configured to engage an interior surface of the opening in the plunger body head; and
a base comprising a plurality of proximally-extending ribs configured to engage a distal-facing surface of the plunger body head.
